# FASCICULE DE BREVET EUROPEEN

(11) **EP 2 950 887 B1**
(45) Date de publication et mention de la délivrance du brevet: **29.11.2017**
(21) Numéro de dépôt: 14702000.2
(22) Date de dépôt: 29.01.2014
(51) Int. Cl.: A61Q 19/00, A61K 8/97

(54) **UTILISATION DE CELLULES VÉGÉTALES D'IRIDACÉE ÉLICITÉES DANS LE TRAITEMENT DES PEAUX SENSIBLES**
VERWENDUNG VON BEHANDELTEN IRIDACAE ZELLEN ZUR BEHANDLUNG EMPFINDLICHER HAUT
USE OF ELICITED IRIDACAE CELL FOR SENSITIVE SKIN TREATMENT

(30) Priorité: 29.01.2013 FR 1350758
(43) Date de publication de la demande: 09.12.2015
(73) Titulaire: L'Oreal, S.A., 75008 Paris (FR)
(72) Inventeur: MARTIN, Richard, F-37210 Rochecorbon (FR); HILAIRE, Pascal, F-37210 Vouvray (FR)
(74) Mandataire: Lavoix
(86) Numéro de dépôt international: PCT/EP2014/051684
(87) Numéro de publication internationale: WO 2014/118207

(56) Documents cités:
- EP-A1- 0 723 774
- EP-A1- 1 174 120
- EP-A1- 1 452 167
- WO-A1-97/09056
- FR-A1- 2 738 486
- JP-A- 2012 116 766

## Description

La présente invention concerne le traitement cosmétique ou pharmaceutique des peaux sensibles et des désordres associés à un excès de synthèse et/ou de libération du peptide dérivé de la calcitonine (connu sous le nom de Calcitonine Gene Related Peptide ou CGRP).

Le CGRP est un élément chimique polypeptidique élaboré et libéré par une terminaison nerveuse. La localisation du CGRP étant spécifique des fibres nerveuses sensitives (fibres C), de très nombreux organes ou tissus reçoivent des afférences de neurones à CGRP, tels que les glandes salivaires, l'estomac, le pancréas, l'intestin, le système cardio-vasculaire, la glande thyroïde ou la peau.

De par cette distribution ubiquitaire du CGRP, de très nombreux désordres sont associés à un excès de synthèse et/ou de libération du CGRP. C'est le cas notamment de maladies respiratoires et inflammatoires, de maladies allergiques et de désordres cutanés tels que des affections dermatologiques incluant l'eczéma, le prurigo ou la rosacée.

L'utilisation d'antagonistes du CGRP constitue un traitement efficace dans toutes les affections citées ci-dessus.

Par ailleurs, la Demanderesse a démontré que l'une des caractéristiques essentielles des peaux sensibles était liée à la libération du CGRP. Par conséquent, l'utilisation d'antagonistes du CGRP peut également permettre d'obtenir un effet préventif et/ou curatif des peaux sensibles.

Par "antagoniste du CGRP", on entend ici tout composé susceptible d'inhiber partiellement, voire totalement, l'effet biologique du CGRP.

Parmi les antagonistes du CGRP connus, on peut citer par exemple le CGRP 8-37 (séquence des acides aminés 8 à 37 de la partie N-terminale du CGRP), ou encore les anticorps anti-CGRP.

Par ailleurs, la Demanderesse a montré dans les demandes WO 97/09056 et FR 2 738 486 qu'un extrait d'au moins une Iridacée était capable de diminuer la vasodilatation induite par la capsaïcine et/ou par une stimulation électrique antidromique (appliquée sur un nerf afférent) et/ou de provoquer une inhibition de la libération de CGRP par les fibres nerveuses sensitives et/ou de provoquer une inhibition de la contraction du muscle lisse du vas deferens induite par le CGRP, et pouvait donc être utilisé comme antagoniste du CGRP, en particulier pour traiter les affections citées ci-dessus et les peaux sensibles.

Toutefois, cet extrait ne permettait d'inhiber le CGRP que partiellement, de l'ordre de 70%.

Il existe donc toujours un besoin important de compositions présentant une activité antagoniste du CGRP plus importante.

Par ailleurs, l'extrait décrit dans la demande FR 2 738 486 pouvait contenir des composés délétères à l'égard de l'activité antagoniste du CGRP et les molécules responsables de cette activité antagoniste n'étaient pas excrétées dans le milieu de culture, réduisant donc la productivité de la culture.

Il existe donc encore un besoin important de compositions présentant une activité antagoniste du CGRP pouvant être obtenues avec une forte productivité.

La présente invention résulte de la découverte inattendue, par les inventeurs, qu'un extrait de *Lactobacillus delbrueckii spp lactis* agit comme éliciteur sur les cellules végétales d'iris. En effet, lorsqu'il est ajouté à une culture de cellules dédifférenciées d'iris, cet extrait permet une surexpression de l'activité antagoniste du CGRP à la fois intracellulaire et extracellulaire des cellules d'iris, induisant une inhibition du CGRP maximalisée, de l'ordre de 100%. De plus, l'excrétion, dans le milieu extérieur, des molécules responsables de cette activité antagoniste, permet d'augmenter de façon importante la productivité de la culture.

La présente invention concerne donc l'utilisation d'un extrait de *Lactobacillus delbrueckii* pour éliciter une cellule végétale d'une Iridacée du genre Romulea, Crocus, Iris, Gladiolus, Sisyrichium ou Hermodactylus. Elle concerne également l'utilisation d'un extrait de *Lactobacillus delbrueckii* pour induire la production d'un antagoniste du CGRP par une cellule végétale d'une Iridacée.

La présente invention a également pour objet un procédé d'élicitation desdites cellules végétales d'une Iridacée comprenant la mise en contact desdites cellules végétales d'une Iridacée avec *Lactobacillus delbrueckii* sous forme d'extrait dans des conditions appropriées pour induire l'expression d'une activité antagoniste du CGRP.

Elle a également trait à un procédé de préparation d'une composition ayant une activité antagoniste du CGRP comprenant les étapes consistant à :
a) mettre en contact, dans un milieu de culture, lesdites cellules végétales d'une Iridacée avec *Lactobacillus delbrueckii* sous forme d'extrait dans des conditions appropriées pour induire l'expression d'une activité antagoniste du CGRP,
b) éventuellement séparer les cellules végétales cultivées à l'étape a) du milieu de culture, et
c) récupérer une composition ayant une activité antagoniste du CGRP.

La présente invention concerne également une composition ayant une activité antagoniste du CGRP susceptible d'être obtenue par ce procédé de préparation.

Elle a également pour objet une composition cosmétique comprenant une composition ayant une activité antagoniste du CGRP susceptible d'être obtenue par ce procédé de préparation, et un véhicule cosmétiquement acceptable.

Un autre objet de la présente invention concerne l'utilisation de cette composition cosmétique pour traiter les peaux sensibles ou dans le traitement antipelliculaire.

La présente invention concerne enfin une composition ayant une activité antagoniste du CGRP susceptible d'être obtenue par le procédé de préparation ci-dessus pour son utilisation dans le traitement des désordres associés à un excès de synthèse et/ou de libération du CGRP.

### Description détaillée de l'invention

### Activité antagoniste du CGRP

Dans le contexte de l'invention, le terme "peptide dérivé de la calcitonine" "Calcitonine Gene Related Peptide" ou "CGRP" fait référence à un peptide membre de la famille de la calcitonine produit dans les neurones centraux et périphériques et qui existe, chez l'homme, sous deux formes, l'α-CGRP et le β-CGRP. Le CGRP agit via un récepteur hétéromère composé d'un récepteur couplé à une protéine G, appelé "calcitonin receptor-like receptor" ou CALCRL, et une protéine modifiant l'activité du récepteur ou RAMP.

Par "antagoniste du CGRP", on entend ici tout composé susceptible d'inhiber partiellement, voire totalement, l'effet biologique du CGRP. De préférence, un antagoniste du CGRP ou un composé ayant une activité antagoniste du CGRP selon l'invention est capable de produire une inhibition de la fixation réceptorielle du CGRP ou de produire une inhibition de la synthèse et/ou de la libération de CGRP par les fibres nerveux sensitives. De manière particulièrement préférée, un antagoniste du CGRP ou un composé ayant une activité antagoniste du CGRP selon l'invention est capable de produire une inhibition de la fixation réceptorielle du CGRP. De manière davantage préféré, un antagoniste du CGRP ou un composé ayant une activité antagoniste du CGRP selon l'invention a une affinité pour les récepteurs au CGRP.

### Extrait de Lactobacillus delbrueckii

Par *"Lactobacillus delbrueckii"*, on entend ici un bacille à Gram positif du genre *Lactobacillus,* non-mobile, capable de fermenter les substrats glucidiques en acide lactique dans des conditions anaérobies. Comme il est bien connu de l'homme du métier, l'espèce *Lactobacillus delbrueckii* inclut 4 sous-espèces : *Lactobacillus delbrueckii spp bulgaricus, Lactobacillus delbrueckii spp lactis, Lactobacillus delbrueckii delbrueckii* et *Lactobacillus delbrueckii indicus.*

De préférence, la sous-espèce de *Lactobacillus delbrueckii* utilisée dans le cadre de l'invention est *Lactobacillus delbrueckii spp lactis. Lactobacillus delbrueckii spp lactis* inclut en particulier les souches ATCC 4797, DSM 20076, NCDO 299, NCIB 7854, DSM 7290 et NCC 88. De préférence, la souche de *Lactobacillus delbrueckii spp lactis* utilisée dans le cadre de l'invention est la souche ATCC 4797.

La bactérie *Lactobacillus delbrueckii* utilisée dans le cadre de l'invention peut être mise en oeuvre sous une forme d'extraits de bactérie inactivée ou morte. De préférence, la bactérie *Lactobacillus delbrueckii* est mise en oeuvre sous forme inactivée ou morte.

Au sens de l'invention, un microorganisme "inactivé" est un microorganisme qui n'est plus capable, temporairement ou définitivement, de former des colonies en culture.

Au sens de l'invention, un microorganisme "mort" est un microorganisme qui n'est plus capable, définitivement, de former des colonies en culture. Les microorganismes morts ou inactivés peuvent avoir les membranes cellulaires intactes ou rompues. Ainsi le terme "inactivé" désigne également les extraits et lysats de microorganismes comme détaillés ci-après. L'obtention de microorganismes morts ou inactivés peut être effectuée par toute méthode connue de l'homme du métier.

Selon un mode de réalisation avantageux, les bactéries *Lactobacillus delbrueckii* mises en oeuvre selon l'invention sont au moins en partie inactivées ou mortes.

Par "bactéries *Lactobacillus delbrueckii* au moins en partie inactivées", on désigne une préparation de bactéries *Lactobacillus delbrueckii* conformes à l'invention comprenant au moins 80 %, en particulier au moins 85 %, plus particulièrement au moins 90 %, voire au moins 95 %, ou au moins 99 % de bactéries *Lactobacillus delbrueckii* inactivées exprimées en unité formant colonie (ufc) par rapport à la totalité des bactéries *Lactobacillus delbrueckii* vivantes non inactivées contenues dans la préparation initiale avant d'être soumise à un procédé d'inactivation. Le taux d'inactivation obtenu dépend des conditions d'application de la méthode d'inactivation qui sont ajustées par l'homme du métier selon le taux d'inactivation à obtenir. Selon un mode de réalisation, l'invention comprend la mise en oeuvre d'une préparation comprenant 100 % de bactéries *Lactobacillus delbrueckii* inactivées.

Des bactéries *Lactobacillus delbrueckii* inactivées convenant à l'invention peuvent être préparées par irradiation, inactivation thermique ou lyophilisation d'une préparation de bactéries *Lactobacillus delbrueckii.* Ces méthodes sont connues de l'homme du métier.

Les bactéries *Lactobacillus delbrueckii* selon l'invention sont mis en oeuvre sous forme d'extraits ou de lysats comprenant des fractions et/ou des métabolites de ces bactéries.

Au sens de l'invention, le terme "fraction" désigne un fragment de ladite bactérie doté d'une efficacité d'élicitation par analogie à ladite bactérie entière.

Au sens de l'invention, le terme "métabolite" désigne toute substance issue du métabolisme des bactéries, et notamment secrétée par les bactéries considérées selon l'invention et dotée également d'une efficacité d'élicitation.

Un extrait ou lysat convenant à l'invention peut être préparé à partir de bactéries, en fin de phase de croissance.

Selon l'invention, la bactérie *Lactobacillus delbrueckii* convenant à l'invention est préparée sous forme d'un extrait.

Un extrait au sens de l'invention désigne communément un matériau obtenu à l'issue de la destruction ou dissolution de cellules biologiques par un phénomène dit de lyse cellulaire provoquant ainsi la libération des constituants biologiques intracellulaires naturellement contenus dans les cellules du microorganisme considéré. Au sens de la présente invention, le terme "extrait" est utilisé indifféremment pour désigner l'intégralité du lysat obtenu par lyse du microorganisme concerné ou seulement une fraction de celui-ci. L'extrait mis en oeuvre est donc formé en tout ou partie des constituants biologiques intracellulaires et des constituants des parois et membranes cellulaires de la bactérie *Lactobacillus delbrueckii.* Avantageusement, un extrait utilisé pour l'invention peut être l'intégralité de l'extrait obtenu par lyse de la bactérie *Lactobacillus delbrueckii.*

Cette lyse cellulaire peut être accomplie par différentes technologies, telles que par exemple un choc osmotique, un choc thermique, par ultrasons, ou encore sous contrainte mécanique de type centrifugation ou pression.

Plus particulièrement, cet extrait peut être obtenu selon le protocole suivant : l'extrait est obtenu par passage d'un milieu comprenant les bactéries *Lactobacillus delbrueckii* à la Presse de French, suivie de préférence par des filtrations en cascade, et de préférence d'une lyophilisation.

Un extrait peut être mis en oeuvre sous différentes formes, tel que sous la forme d'une solution ou sous une forme pulvérulente.

Les présents inventeurs ont montré en particulier qu'un extrait de *Lactobacillus delbrueckii spp lactis,* obtenu par la technique décrite ci-dessus, agissait comme éliciteur sur les cellules végétales d'iris, alors que d'autres bactéries à Gram positif n'avaient pas cette propriété.

### Cellule végétale d'Iridacée

La famille des Iridacées (ou Iridées) compte environ 750 espèces. Les plantes de cette famille sont surtout utilisées pour leurs propriétés aromatiques et ornementales. Les cellules végétales utilisées dans le cadre de l'invention, proviennent des genres Romulea, Crocus, Iris, Gladiolus, Sisyrinchium et Hermodactylus. De préférence, les cellules végétales utilisées dans le cadre de l'invention sont des cellules végétales d'Iris.

Les cellules végétales utilisées dans le cadre de l'invention peuvent plus particulièrement provenir *d'Iris germanica, d'Iris florentina, d'Iris pallida,* de *Crocus versicolor,* de *Romulea bulbucodium* ou encore de *Gladiolus communis.* De préférence, les cellules végétales utilisées dans le cadre de l'invention proviennent d'*Iris pallida.*

De manière particulièrement préférée, la cellule végétale utilisée dans le cadre de l'invention est une cellule dédifférenciée d'une Iridacée, telle que définie ci-dessus.

Par "cellule végétale dédifférenciée", on entend ici toute cellule végétale ne présentant aucun des caractères d'une spécialisation particulière et capable de vivre par elle-même et non en dépendance avec d'autres cellules. Ces cellules végétales dédifférenciées sont éventuellement aptes, sous l'effet d'une induction, à toute différenciation conforme à leur génome.

Les cellules végétales utilisées dans le cadre de l'invention sont de préférence des cellules végétales cultivées *in vitro.*

La pression de sélection imposée par les conditions physico-chimiques lors de la culture des cellules végétales *in vitro* permet en effet d'obtenir un matériel végétal standardisé et disponible tout au long de l'année contrairement aux plantes cultivées *in vivo.*

### Procédé d'élicitation

La présente invention concerne l'utilisation de *Lactobacillus delbrueckii,* plus particulièrement d'un extrait de *Lactobacillus delbrueckii,* tel que défini dans la section *"Lactobacillus delbrueckii"* ci-dessus, pour éliciter une cellule végétale d'une Iridacée, telle que définie dans la section *"Cellule végétale d'Iridacée"* ci-dessus.

Elle concerne également un procédé d'élicitation de cellules végétales d'une Iridacée comprenant la mise en contact de cellules végétales d'une Iridacée, telle que définie dans la section *"Cellule végétale d'Iridacée"* ci-dessus, avec *Lactobacillus delbrueckii,* de préférence avec un extrait de *Lactobacillus delbrueckii spp lactis,* tel que défini dans la section *"Lactobacillus delbrueckii"* ci-dessus, dans des conditions appropriées pour induire l'expression d'une activité antagoniste du CGRP, telle que définie dans la section *"Antagoniste du CGRP"* ci-dessus.

Par "élicitation", on entend ici le fait d'induire, par un éliciteur exogène, chez un autre organisme ou cellule, une voie métabolique peu exprimée ou de réveiller, chez un autre organisme ou cellule, des voies métaboliques silencieuses.

De préférence, par "élictation", on entend ici la stimulation, par un éliciteur exogène, de l'activité antagoniste du CGRP, telle que définie dans la section *"Antagoniste du CGRP"* ci-dessus, dans un autre organisme ou cellule.

Par "éliciteurs", on entend ici des molécules ou organismes capables d'induire, chez un autre organisme, une voie de métabolique peu exprimée ou de réveiller, chez un autre organisme, des voies métaboliques silencieuses. Un grand nombre d'éliciteurs sont connus de l'homme du métier et incluent des éliciteurs biotiques, tels que le jasmonate et ses dérivés, et des éliciteurs abiotiques tels que la température, le pH ou un choc osmotique.

Par "mettre en contact", on entend ici incuber, dans un même milieu de culture les cellules végétales et la bactérie *Lactobacillus delbrueckii,* selon l'invention l'extrait de *Lactobacillus delbrueckii.*

Par "conditions appropriées pour induire l'expression d'une activité antagoniste du CGRP", on entend ici des conditions de durée et de température, et/ou une concentration en bactérie *Lactobacillus delbrueckii,* permettant d'induire l'expression d'une activité antagoniste du CGRP telle que définie à la section *"Antagoniste du CGRP"* ci-dessus, de préférence permettant d'induire l'expression d'une activité agoniste du CGRP entrainant une inhibition de plus de 80%, de préférence de plus de 90%, de préférence de plus 95%, 96%, 97%, 98%, 99%, de manière préférée entre toutes de 100% du récepteur au CGRP. De telles conditions peuvent être déterminées par l'homme du métier par des techniques de routine.

En effet, les techniques permettant de déterminer l'expression d'une activité antagoniste du CGRP sont bien connues de l'homme du métier et sont décrites par exemple dans "Calcitonin gene-related peptide (CGRP) and its role in hypertension", Sarah-Jane Smillie, Susan D. Brain. BHF Centre of Cardiovascular Excellence and Centre for Integrative Biomedicine, Cardiovascular Division, Franklin-Wilkins Building, Waterloo Campus, King's College London, London SE1 9NH, UK.

Typiquement, l'expression d'une activité antagoniste du CGRP peut être mise en évidence comme suit : des cellules HEK transfectées stablement par le récepteur hCALCRL et la protéine associée hRAMP1 et exprimant le gène rapporteur de la β-lactamase sous le contrôle de l'élément de réponse à l'AMP cyclique (CRE) sont cultivées, de préférence en milieu DMEM + sérum, à 37°C et 5% CO₂. Les cellules sont ensuite traitées par le ligand de référence CGRP, par exemple à son EC80 (typiquement 0.2 nM final) et différentes doses des compositions candidates. Après incubation, de préférence entre 2 et 4 heures, par exemple 3 heures, d'incubation, à 37°C 5% CO₂, le substrat de la β-lactamase est ajouté. Après incubation, par exemple pendant 2 heures, typiquement à température ambiante, la dégradation du substrat de la β-lactamase est mesurée, par exemple au spectrophotomètre.

De préférence, dans le procédé d'élicitation selon l'invention, les cellules végétales d'une Iridacée, telle que définie dans la section *"Cellule végétale d'Iridacée"* ci-dessus, sont mises en contact avec *Lactobacillus delbrueckii,* de préférence avec un extrait de *Lactobacillus delbrueckii spp lactis,* tel que défini dans la section *"Lactobacillus delbrueckii"* ci-dessus, à une concentration de 200 µg d'extrait / litre de culture pendant 7 jours à une température de 26°C.

### Préparation d'une composition ayant une activité antagoniste du CGRP

La présente invention concerne également l'utilisation de *Lactobacillus delbrueckii,* sous forme d'un extrait de *Lactobacillus delbrueckii,* tel que défini dans la section *"Lactobacillus delbrueckii"* ci-dessus, pour induire la production d'un antagoniste du CGRP, tel que défini dans la section *"Antagoniste du CGRP"* ci-dessus, par une cellule végétale d'une Iridacée, telle que définie dans la section *"Cellule végétale d'Iridacée"* ci-dessus.

La présente invention concerne également un procédé de préparation d'une composition ayant une activité antagoniste du CGRP comprenant les étapes consistant à :
a) mettre en contact, dans un milieu de culture, des cellules végétales d'une Iridacée, telle que définie dans la section *"Cellule végétale d'Iridacée"* ci-dessus, avec *Lactobacillus delbrueckii,* sous forme d'un extrait de *Lactobacillus delbrueckii,* tel que défini dans la section *"Lactobacillus delbrueckii"* ci-dessus, dans des conditions appropriées pour induire l'expression d'une activité antagoniste du CGRP, telles que définies dans la section *"Procédé d'élicitation"* ci-dessus,
b) éventuellement séparer les cellules végétales cultivées à l'étape a) du milieu de culture, et
c) récupérer une composition ayant une activité antagoniste du CGRP.

Par "mettre en contact", on entend ici incuber, dans un même milieu de culture les cellules végétales et la bactérie *Lactobacillus delbrueckii,* sous forme de l'extrait de *Lactobacillus delbrueckii.*

Par "milieu de culture", on entend ici une solution comprenant tous les nutriments nécessaires aux cellules végétales pour leur survie et leur croissance. De tels milieux de culture sont bien connus de l'homme du métier et sont par exemple décrits dans "Plant Culture Media: Formulation and Uses, Edwin F.George, DavidJ.M.Puttock and Heather J.George". Des exemples de milieux de culture appropriés pour la mise en oeuvre de l'invention incluent le milieu décrit dans Murashige and Skoog (1962) *Physiol. Plant. 15:4*73-497.

Par "conditions appropriées pour induire l'expression d'une activité antagoniste du CGRP", on entend ici les mêmes conditions que celles décrites dans la section *"Procédé d'élicitation"* ci-dessus.

Toute technique de séparation conventionnelle peut être utilisée pour séparer les cellules végétales du milieu de culture à l'étape optionnelle b). De telles techniques incluent par exemple la centrifugation, la filtration ou la décantation.

Par "composition ayant une activité antagoniste du CGRP", on entend ici un mélange comprenant des molécules ayant une activité antagoniste du CGRP, telle que définie dans la section *"Antagoniste du CGRP"* ci-dessus, produites par les cellules végétales suites à leur mise en contact avec *Lactobacillus delbrueckii* à l'étape a) du procédé selon l'invention. Une telle composition peut donc contenir d'autres composés que ceux ayant une activité antagoniste du CGRP, en particulier d'autres composés issus de la cellule végétale et/ou de *Lactobacillus delbrueckii* et/ou du milieu de culture.

La nature des molécules ayant cette activité antagoniste du CGRP n'a pas encore été déterminée. Toutefois, il a été démontré par la Demanderesse que ces molécules ayant une activité antagoniste sont uniquement exprimées par les cellules végétales cultivées, et non par *Lactobacillus delbrueckii.*

Dans un mode de réalisation particulier de l'invention, la composition ayant une activité antagoniste du CGRP récupérée à l'étape c) est un extrait des cellules végétales séparées à l'étape b).

Toute méthode d'extraction connue de l'homme du métier peut être utilisée pour obtenir un extrait des cellules végétales séparées à l'étape b). On peut en particulier citer les extraits alcooliques, notamment éthanoliques, ou les extraits hydroalcooliques. L'extrait de cellules végétales peut également être un extrait aqueux, typiquement préparé par remise en suspension des cellules végétales séparées dans de l'eau osmosée, de préférence dans une quantité équivalente au volume de la culture de départ, suivie de préférence d'un passage en homogénéisateur haute pression (HHP) typiquement à 1250 Bar, centrifugation, de préférence à 6000 g, pendant 20 min à 4°C, filtration, par exemple sur filtre d'épaisseur tel que le filtre GFD® et/ou sur filtre GFF ("*Gravity flow fiber"*), et congélation, par exemple à -20°C.

La Demanderesse a démontré que la mise en contact de *Lactobacillus delbrueckii* permettait d'induire la sécrétion dans le milieu extérieur des molécules responsables de l'activité antagoniste du CGRP produite par les cellules végétales.

Dans un mode de réalisation particulier de l'invention, la composition ayant une activité antagoniste du CGRP récupérée à l'étape c) est donc issue du milieu de culture séparé à l'étape b). Ce milieu de culture peut être lyophilisé, atomisé ou concentré en l'état ou subir un fractionnement sur tamis moléculaire de type filtration tangentielle puis être traité suivant les techniques ci-avant décrites.

Dans un autre mode de réalisation particulier de l'invention, le procédé de préparation d'une composition ayant une activité antagoniste du CGRP ne comprend pas d'étape de séparation des cellules végétales cultivées à l'étape a) du milieu de culture, et la composition ayant une activité antagoniste du CGRP récupérée à l'étape c) comprend donc à la fois le milieu de culture et les cellules végétales cultivées et élicitées à l'étape a).

Par "composition ayant une activité antagoniste du CGRP", on entend ici un mélange comprenant des molécules ayant une activité antagoniste du CGRP, telle que définie dans la section *"Antagoniste du CGRP"* ci-dessus, produites par les cellules végétales suites à leur mise en contact avec *Lactobacillus delbrueckii* à l'étape a) du procédé selon l'invention. Une telle composition peut donc contenir d'autres composés que ceux ayant une activité antagoniste du CGRP, en particulier d'autres composés issus de la cellule végétale et/ou de *Lactobacillus delbrueckii* et/ou du milieu de culture.

La présente invention concerne donc également une composition ayant une activité antagoniste du CGRP susceptible d'être obtenue, de préférence obtenue, par le procédé de préparation selon l'invention décrit ci-dessus. De préférence, cette composition ayant une activité antagoniste du CGRP comprend des composés issus de *Lactobacillus delbrueckii.*

La nature des molécules ayant cette activité antagoniste du CGRP n'a pas encore été déterminée. Toutefois, il a été démontré par la Demanderesse que ces molécules ayant une activité antagoniste sont uniquement exprimées par les cellules végétales cultivées, et non par *Lactobacillus delbrueckii.*

### Composition cosmétique et pharmaceutique

La présente invention concerne également une composition cosmétique ou pharmaceutique comprenant une composition ayant une activité antagoniste du CGRP telle que définie à la section *"Préparation d'une composition ayant une activité antagoniste du CGRP"* ci-dessus, et un véhicule cosmétiquement ou pharmaceutiquement acceptable.

Par "véhicule cosmétiquement acceptable", on entend ici un milieu sans odeur ou aspect désagréable, et qui ne génère pas de picotement, de tiraillement ou de rougeur inacceptable pour l'utilisateur, lors de son administration, en particulier lors de son application topique sur la peau ou ses annexes.

Par "véhicule pharmaceutiquement acceptable", on entend ici tout solvant, milieu de dispersion, agent retardant l'absorption, etc., qui ne produit pas de réaction secondaire, par exemple allergique, chez l'humain ou l'animal. Les véhicules pharmaceutiquement acceptables sont bien connus de l'homme du métier, et incluent ceux décrits dans "Remington's Pharmaceutical Sciences" (Mack Publishing Company, Easton, USA, 1985).

Le véhicule cosmétiquement ou pharmaceutiquement acceptable sera adapté à la voie d'administration de la composition cosmétique ou pharmaceutique, en particulier à la nature du support sur lequel doit être appliquée la composition cosmétique ou pharmaceutique, ainsi qu'à la forme sous laquelle la composition cosmétique ou pharmaceutique est destinée à être conditionnée, notamment solide ou fluide à température ambiante et pression atmosphérique.

Selon l'invention, la composition ayant une activité antagoniste du CGRP, telle que définie à la section *"Préparation d'une composition ayant une activité antagoniste du CGRP"* ci-dessus, est utilisée en une quantité représentant de 0,001 % à 20% du poids total de la composition cosmétique ou pharmaceutique et préférentiellement en une quantité représentant de 0,1% à 10% du poids total de la composition cosmétique ou pharmaceutique.

La composition cosmétique ou pharmaceutique selon l'invention peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition cosmétique ou pharmaceutique selon l'invention peut se présenter sous toutes les formes galéniques normalement utilisées.

Pour une application topique sur la peau, la composition cosmétique ou pharmaceutique selon l'invention peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions cosmétiques ou pharmaceutiques sont préparées selon les méthodes usuelles.

Elles peuvent également être utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition cosmétique ou pharmaceutique selon l'invention peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions cosmétiques ou pharmaceutiques selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes antisolaires), des fonds de teint fluides, des laits de démaquillage, des laits corporels de protection ou de soin, des laits antisolaires, des lotions, gels ou mousses pour le soin de la peau, comme des lotions de nettoyage, des lotions antisolaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions antidouleur, des compositions pour traiter certaines maladies de la peau comme l'eczéma, les ulcères de la jambe, la rosacée, le psoriasis, les lichens, les prurits sévères, les pustules ou les vergetures.

Les compositions cosmétiques ou pharmaceutiques selon l'invention peuvent également consister en des préparations solides constituant des savons ou des pains de nettoyage.

Les compositions cosmétiques ou pharmaceutiques selon l'invention peuvent aussi être conditionnées sous forme de composition pour aérosol comprenant également un agent propulseur sous pression.

La composition ayant une activité antagoniste du CGRP utilisée selon l'invention peut aussi être incorporée dans diverses compositions pour soins capillaires, et notamment des shampoings, des lotions de mise en plis, des lotions traitantes, des crèmes ou des gels coiffants, des compositions de teintures (notamment teintures d'oxydation) éventuellement sous forme de shampoings colorants, des lotions restructurantes pour les cheveux, des compositions de permanente (notamment des compositions pour le premier temps d'une permanente), des lotions ou des gels antichute, des shampoings antiparasitaires, etc.

Les compositions cosmétiques ou pharmaceutiques selon l'invention peuvent aussi être à usage bucco-dentaire, par exemple une pâte dentifrice. Dans ce cas, elles peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition cosmétique ou pharmaceutique selon l'invention est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents de préférence, dans la composition cosmétique ou pharmaceutique, en une proportion allant de 0,3% à 30% en poids, et de manière davantage préférée de 0,5 à 20% en poids par rapport au poids total de la composition cosmétique ou pharmaceutique. L'émulsion peut, en outre, contenir des vésicules lipidiques.

Lorsque la composition cosmétique ou pharmaceutique selon l'invention est une solution ou un gel huileux, la phase grasse peut représenter plus de 90% du poids total de la composition cosmétique ou pharmaceutique.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 10% du poids total de la composition cosmétique. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcelin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose® 63 par la société Gattefosse.

Comme solvants utilisables dans l'invention, on peut citer les alcools inférieurs, notamment l'éthanol et l'isopropanol, ou le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition cosmétique selon l'invention peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut citer le rétinol (vitamine A) et ses dérivés, le tocophérol (vitamine E) et ses dérivés, les acides gras essentiels, les céramides, les huiles essentielles, l'acide salicylique et ses dérivés.

Selon l'invention, on peut, entre autres, associer au moins une composition ayant une activité antagoniste du CGRP telle que définie à la section *"Préparation d'une composition ayant une activité antagoniste du CGRP'* ci-dessus à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine ;
- les agents antiviraux tels que l'acyclovir ;
- les agents anti-inflammatoires, en particulier les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicalaires ou anti-radicaux libres, tels que l'alpha-tocophérol ou ses esters, les superoxyde dismutases, certains chélatants de métaux ou l'acide ascorbique et ses esters ;
- les agents apaisants tels que les bactéries filamenteuses ou extraits de bactéries filamenteuses comme *Vitreoscilla filiformis* tel que décrit dans le brevet EP 761 204 et commercialisé par Chimex sous la dénomination Mexoryl SBG® ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les vitamines et leurs dérivés ou précurseurs, seuls ou en mélanges ;
- les agents anti-oxydants ;
- les filtres UV hydrophiles ou insolubles dans l'eau et dans les huiles ;
- les filtres UV inorganiques ;
- les agents autobronzants ;
- les agents anti-glycation ;
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents dermorelaxants ;
- les agents tenseurs ;
- les agents matifiants ;
- les agents desquamants ;
- les agents hydratants ;
- les agents agissant sur le métabolisme énergétique des cellules ;
- les agents répulsifs contre les insectes ;
- les antagonistes de substances P ;
- les agents anti-rides ;
- les agents anti-photovieillissement ;
- les agents colorants ;
- les agents nacrants ; et
- les pigments.

Ainsi, selon un mode particulier, l'invention concerne une composition cosmétique ou pharmaceutique comprenant une composition ayant une activité antagoniste du CGRP telle que définie à la section *"Préparation d'une composition ayant une activité antagoniste du CGRP"* ci-dessus, une véhicule cosmétiquement ou pharmaceutiquement acceptable, et au moins un agent choisi parmi les agents antibactériens, antiparasitaires, antifongiques, antiviraux, anti-inflammatoires, antiprurigineux, anesthésiques, kératolytiques, anti-radicaux libres, apaisants, anti-séborrhéiques, antipelliculaires, antiacnéiques et/ou les agents modulant la différenciation et/ou la prolifération et/ou la pigmentation cutanée.

### Utilisation cosmétique

La Demanderesse a démontré que l'une des caractéristiques essentielles des peaux sensibles était liée à la libération de CGRP et donc que l'utilisation de compositions ayant une activité antagoniste du CGRP pouvait permettre d'obtenir un effet préventif et/ou curatif des peaux sensibles.

La présente invention concerne donc l'utilisation d'une composition cosmétique telle que définie dans la section *"Composition cosmétique ou pharmaceutique"* ci-dessus pour traiter les peaux sensibles.

Comme il est rappelé dans la demande européenne EP 0 723 774, les peaux sensibles peuvent être scindées en deux grandes formes cliniques, les peaux irritables et/ou réactives, et les peaux intolérantes.

Une peau irritable et/ou réactive est une peau qui réagit par un prurit, c'est-à-dire par des démangeaisons ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments. En général, ces signes sont associés à une peau hyperséborrhéique ou acnéique avec ou sans dartres, et à un érythème.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à l'eau dure à forte concentration en calcaire et/ou aux variations de température.

La Demanderesse a montré qu'une composition cosmétique comprenant une composition ayant une activité antagoniste du CGRP telle que définie dans la section *"Antagoniste du CGRP"* ci-dessus permettait d'éviter l'irritation et/ou les sensations dysesthésiques et/ou les prurits de la peau et/ou des muqueuses et/ou l'érythème.

Plus précisément, l'invention permet de traiter les symptômes d'origine neurogène, dus à un agent exogène susceptible de modifier les constantes biophysiques et biochimiques du tissu concerné (peau, muqueuses).

Par ailleurs, une peau sensible est une peau qui réagit plus facilement que d'autres à des facteurs extérieurs. Toute irritation d'une peau sensible commence par des signes subjectifs (picotement, échauffement,...) avant d'arriver à l'inflammation.

La présente invention a donc encore pour objet l'utilisation, en particulier cosmétique, d'une composition cosmétique telle que définie dans la section *"Composition cosmétique ou pharmaceutique"* ci-dessus pour prévenir et/ou lutter contre les irritations cutanées et/ou les dartres et/ou les érythèmes et/ou les sensations d'échauffement et/ou de dysesthésie et/ou les prurits de la peau et/ou des muqueuses.

Les cuirs chevelus "sensibles" ont une sémiologie clinique plus univoque : les sensations de prurit et/ou de picotements et/ou d'échauffements sont essentiellement déclenchées par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment observés chez les individus ayant un tel cuir chevelu "sensible".

La présente invention concerne donc également l'utilisation, en particulier cosmétique, d'une composition cosmétique telle que définie dans la section *"Composition cosmétique ou pharmaceutique"* ci-dessus dans le traitement antipelliculaire.

La présente invention concerne également un procédé de traitement cosmétique non-thérapeutique des peaux sensibles, dans laquelle on applique sur la peau, sur le cuir chevelu, et/ou sur les muqueuses, une quantité cosmétiquement efficace d'une composition cosmétique telle que définie dans la section *"Composition cosmétique ou pharmaceutique"* ci-dessus.

La présente invention a également pour objet un procédé de traitement antipelliculaire, dans laquelle on applique sur le cuir chevelu une quantité cosmétiquement efficace d'une composition cosmétique telle que définie dans la section *"Composition cosmétique ou pharmaceutique"* ci-dessus.

Par "quantité cosmétiquement efficace", on entend ici une quantité suffisante des agents utilisés dans le cadre de l'invention afin de traiter et/ou prévenir ledit trouble cosmétique, qui ne génère pas d'effets secondaires inacceptables pour l'utilisateur.

Les procédés de traitement cosmétique de l'invention peuvent être mis en oeuvre notamment en appliquant les compositions cosmétiques telles que définies dans la section *"Composition cosmétique ou pharmaceutique"* ci-dessus, selon la technique habituelle de ces compositions. Par exemple : application de crèmes, de gels, de sérums, de lotions, de laits de démaquillage ou de compositions après-solaires sur la peau ou sur les cheveux secs, application d'une lotion pour cheveux sur cheveux mouillés, de shampoings, ou encore application de dentifrice sur les gencives.

### Utilisation thérapeutique

De par la distribution ubiquitaire du CGRP, de très nombreux désordres sont associés à un excès de synthèse et/ou de libération du CGRP. Il s'agit notamment de maladies respiratoires et inflammatoires, des maladies allergiques et désordres cutanés, notamment d'affections dermatologiques telles que l'eczéma, le prurigo ou la rosacée.

La présente invention concerne donc également une composition ayant une activité antagoniste du CGRP, telle que définie dans la section *"Préparation d'une composition ayant une activité antagoniste du CGRP"* ci-dessus, pour son utilisation dans le traitement des désordres associés à un excès de synthèse et/ou de libération du CGRP.

Elle concerne aussi l'utilisation d'une composition ayant une activité antagoniste du CGRP, telle que définie dans la section *"Préparation d'une composition ayant une activité antagoniste du CGRP"* ci-dessus, pour la fabrication d'une composition pharmaceutique destinée à traiter des désordres associés à un excès de synthèse et/ou de libération du CGRP.

Elle concerne également une méthode de traitement de désordres associés à un excès de synthèse et/ou de libération du CGRP chez un sujet, dans laquelle on administre, chez un sujet qui en a besoin, une quantité thérapeutiquement efficace d'une composition pharmaceutique telle que définie dans la section *"Composition cosmétique ou pharmaceutique"* ci-dessus.

Par "quantité thérapeutiquement efficace", on entend ici une quantité suffisante des agents utilisés dans le cadre de l'invention pour traiter et/ou prévenir ladite maladie, à un rapport bénéfice/risque raisonnable acceptable pour n'importe quel traitement médical. Il sera cependant compris que la dose spécifique thérapeutiquement efficace pour un patient particulier dépendra d'une variété de facteurs incluant la maladie à traiter, la gravité de la maladie, l'activité des agents spécifiques employés, la composition spécifique employée, la durée du traitement, l'âge, le poids et la condition du sujet traité, ainsi que les facteurs analogues bien connus du domaine médical.

Les désordres associés à un excès de synthèse et/ou de libération du CGRP sont en particulier choisis dans le groupe constitué des maladies respiratoires et inflammatoires, les maladies allergiques et les maladies dermatologiques telles que l'eczéma, le prurigo, la rosacée, les prurits sévères, l'acné, les ulcères de la jambe, le psoriasis, les pustules et les vergetures.

De préférence, le désordre associé à un excès de synthèse et/ou de libération du CGRP traité dans le cadre de l'invention est une maladie dermatologique telle que l'eczéma, le prurigo, la rosacée, les prurits sévères, l'acné, les ulcères de la jambe, le psoriasis, les pustules et les vergetures. De manière davantage préférée, le désordre associé à un excès de synthèse et/ou de libération du CGRP traité dans le cadre de l'invention est choisi dans le groupe constitué de l'eczéma, le prurigo et la rosacée. De manière préférée entre toutes, le désordre associé à un excès de synthèse et/ou de libération du CGRP traité dans le cadre de l'invention est la rosacée.

Dans la description et dans les exemples suivants, sauf indication contraire, les pourcentages sont des pourcentages en poids et les plages de valeurs libellées sous la forme "entre ... et ..." incluent les bornes inférieure et supérieure précisées.

### Exemples

### Exemple 1 : Préparation de l'éliciteur

Cet exemple montre un procédé de préparation d'un extrait de *Lactobacillus delbrueckii spp lactis* pouvant être utilisé pour mettre en oeuvre l'invention.

Le *Lactobacillus delbrueckii spp lactis* est cultivé sur milieu traditionnel appelé MRS.

En fin de croissance, les cellules sont soumises à un passage à la Presse de French. Après filtration en cascade, l'extrait est lyophilisé.

### Exemple 2 : Préparation d'extraits de cellules végétales dédifférenciées d'iris

Cet exemple montre un procédé de préparation d'un extrait de cellules végétales dédifférenciées d'iris pouvant être utilisé pour mettre en oeuvre l'invention.

### a) Culture des cellules végétales dédifférenciées d'iris

- *Milieu de culture :* Murashige & Skoog Acide Naphtalène Acétique (ANA) 2 et Kinétine 0,6, saccharose 20 g/l.
- *Culture en Erlenmeyer :* Volume de culture de 400 mL. La culture est réalisée à 26°C, sous agitation de 100 rpm pendant 15 jours.
- *Culture en fermenteur de 10 L :* Fermenteur de 10 litres efficaces, pH non régulé, pO₂ 15% régulée par air. La culture est réalisée à 26°C sous agitation de 100 rpm (Rushton).

En 14 jours de Batch, 120 g de poids frais de cellules / litre soit 8 g de poids sec / litre sont obtenus.

7 jours après l'ensemencement du fermenteur avec la préculture de cellules d'*Iris pallida,* l'éliciteur en solution aqueuse est introduit stérilement dans le fermenteur. La culture est maintenue dans les mêmes conditions pendant 7 jours.

### b) Extraction des cellules végétales dédifférenciées d'iris produites en a)

La culture est filtrée sur filtre Nylon de porosité de 50 µm pour récupérer la biomasse et éliminé le milieu de culture conditionné.

La biomasse est remise en suspension dans de l'eau osmosée, à une quantité équivalente à la culture de départ.

La suspension est passée en Homogénéisateur Haute Pression (HHP) sur microfluidics à 1250 Bar, puis centrifugée à 6000 g pendant 20 min, à 4°C, et filtrée sur GFD et GFF.

Le produit final est ensuite congelé à -20 °C et lyophilisé.

Entre 2,5 et 3,5 g d'extrait / 100 g de cellules fraiches sont ainsi obtenus, soit entre 3 et 4 g/L de culture.

### Exemple 3 : Mise en évidence de l'effet éliciteur de Lactobacillus delbrueckii

Cet exemple montre que l'utilisation d'un extrait de *Lactobacillus delbrueckii spp lactis* permet d'induire l'expression et la sécrétion d'une activité antagoniste du CGRP par des cellules végétales dédifférenciées d'iris.

### Matériel et méthodes

Les extraits d'iris, préparés selon l'Exemple 2, mis en contact ou non avec différentes doses d'extraits de *Lactobacillus delbrueckii spp lactis* préparés selon l'Exemple 1, ont été testés sur la lignée cellulaire HEK transfectée stablement par le récepteur hCALCRL et la protéine associée hRAMP1. Cette lignée exprime le gène rapporteur de la β-lactamase sous le contrôle de l'élément de réponse à l'AMP cyclique (CRE).

Composition du milieu de culture :
- 500 mL milieu DMEM 4,5 g/L (Glucose)
- 50 mL sérum dialysé
- 12,5 mL d'Hepes (1M)
- 5 mL de peni/Strepto/fungizone (100X)

Les extraits ont été dilués extemporanément dans 1 mL de milieu de culture complété à 1 % de DMSO et 0,1% d'acide pluronique, soit une concentration de 20 mg/ml initiale.

Pour certains échantillons, la solubilisation n'a pas été complète, et a donc été scorée visuellement:
- « OK » : solubilisation totale.
- « Suspension» : mélange homogène de petites particules de même taille.
- « Particulaire» : mélange non homogène de particules de tailles différentes.

Les cellules ont été ensemencées la veille en plaques 384 puits et incubées à 37 °C et 5 % CO₂.

Après 24 heures, les cellules ont été traitées par le ligand de référence CGRP à son EC80 (0,2 nM final) et les doses réponses des extraits à tester. Les extraits ont été préalablement dilués en dose-réponse sur 10 points, à 2000 µg/mL final pour la première concentration, et des dilutions sérielles au ¼.

Après 3 heures d'incubation à 37°C 5% CO₂, le substrat de la β-lactamase a été ajouté.

Après 2 heures d'incubation à température ambiante, les plaques ont été lues au spectrophotomètre.

Les données ont été normalisées entre 0% et 100 % d'activité, grâce aux témoins inclus dans la plaque. Les courbes de dose-réponse ont été tracées à l'aide du logiciel XLfit, les IC50 sont exprimées en µg/ml.

### Résultats

Le **tableau 1** ci-dessous présente les résultats obtenus.

**Tableau 1 : Activité antagoniste du CGRP exprimée par les cellules végétales d'iris mises en contact avec L. delbrueckii**

| **Source** | **Lot** | **Concentration *L. delbrueckii*** | **IC50 (µg/mL)** | **% inhibition** | **Solubilisation** |
|---|---|---|---|---|---|
| Milieu conditionné | 1 | 0 | 75 | 96 | particulaire |
| Milieu conditionné | 2 | 0 | 10 | 94 | particulaire |
| Milieu conditionné | 1 | 0,1% | 35 | **97** | particulaire |
| Milieu conditionné | 2 | 0,1% | 1,8 | **112** | particulaire |
| Milieu conditionné | 1 | 0,15% | 30 | **104** | particulaire |
| Milieu conditionné | 2 | 0,15% | 39 | 95 | particulaire |
| Milieu conditionné | 1 | 0,20% | 2,7 | **97** | particulaire |
| Milieu conditionné | 2 | 0,20% | 1,1 | 90 | suspension |
| Milieu vierge | | 0 | N/A | N/A | OK |
| Milieu vierge | | 0,1% | N/A | N/A | OK |
| Milieu vierge | | 0,15% | N/A | N/A | OK |
| Milieu vierge | | 0,20% | N/A | N/A | OK |
| Cellules végétales dédifférenciées | 1 | 0 | 1,4 | 112 | particulaire |
| Cellules végétales dédifférenciées | 2 | 0 | 2,9 | 105 | suspension |
| Cellules végétales dédifférenciées | 1 | 0,1% | 4,6 | 91 | suspension |
| Cellules | 2 | 0,1% | 4,1 | **97** | particulaire |
| végétales dédifférenciées végétales | | | | | |
| Cellules végétales dédifférenciées | 1 | 0,15% | 4,9 | **96** | suspension |
| Cellules végétales dédifférenciées | 2 | 0,15% | 5,6 | **105** | suspension |
| Cellules végétales dédifférenciées | 1 | 0,20% | 0,5 | **111** | particulaire |
| Cellules végétales dédifférenciées | 2 | 0,20% | 0,6 | **107** | particulaire |

Le milieu conditionné est un milieu qui a été en contact avec les cellules végétales.

Le milieu vierge est le milieu avant ensemencement, qui n'a pas été en contact avec les cellules végétales.

Ces résultats montrent donc que l'ajout de l'éliciteur *L. delbrueckii* à la concentration de 0,2% se traduit par un gain d'activité par rapport aux témoins sans éliciteur. En revanche, l'éliciteur *L. delbrueckii* seul n'a pas d'activité.

Ainsi, la Demanderesse a mis au point une technique d'élicitation pour des cellules végétales qui permet à la fois la production intracellulaire mais également l'excrétion du principe actif antagoniste du CGRP de façon extracellulaire augmentant par là même les rendements de production au moins d'un facteur 2.

### Exemple 4 : Comparaison de l'efficacité de Lactobacillus delbrueckii par rapport à d'autres éliciteurs

### Matériel et méthodes

Plusieurs éliciteurs "traditionnels" d'iris ont été testés : le méthyl-jasmonate, la chitine et l'alginate, ainsi que d'autres éliciteurs "non traditionnels" : CaCl₂, *Staphylococcus epidermidis, Lactobacillus delbrueckii,* β-D-gtucane, AlCl₃ et SrCl₂.

Tous les éliciteurs ont été testés à 3 concentrations et en triplicats sur les cellules végétales dédifférenciées d'iris préparées selon l'Exemple 2.

L'activité antagoniste du CGRP a été mesurée comme décrit dans l'Exemple 3.

### Résultats

Le **tableau 2** ci-dessous présente les résultats obtenus.

**Tableau 2 : Activité antagoniste du CGRP exprimée par les cellules végétales d'iris mises en contact avec différents éliciteurs**

| **Eliciteur** | **Concentration** | **Source** | **% inhibition** |
|---|---|---|---|
| - | - | Intracellulaire | 51 |
| - | - | Intracellulaire | 32 |
| Méthyl-jasmonate | 20 mM | Intracellulaire | 57 |
| Méthyl-jasmonate | 20 mM | Extracellulaire | 4 |
| Méthyl-jasmonate | 100 mM | Intracellulaire | 54 |
| Méthyl-jasmonate | 100 mM | Extracellulaire | 5 |
| Méthyl-jasmonate | 500 mM | Intracellulaire | 55 |
| Méthyl-jasmonate | 500 mM | Extracellulaire | 0 |
| Chitine | 0,2 g/L | Intracellulaire | 57 |
| Chitine | 0,2 g/L | Extracellulaire | 10 |
| Chitine | 1 g/L | Intracellulaire | 42 |
| Chitine | 1 g/L | Extracellulaire | 12 |
| Chitine | 5 g/L | Intracellulaire | 37 |
| Chitine | 5 g/L | Extracellulaire | 0 |
| Alginate | 0,2 g/L | Intracellulaire | 60 |
| Alginate | 0,2 g/L | Extracellulaire | 39 |
| Alginate | 1 g/L | Intracellulaire | 53 |
| Alginate | 1 g/L | Extracellulaire | 61 |
| Alginate | 5 g/L | Intracellulaire | 61 |
| Alginate | 5 g/L | Extracellulaire | 16 |
| *S. epidermidis* | 0,20% | Intracellulaire | 54 |
| *S. epidermidis* | 0,20% | Extracellulaire | 9 |
| *S. epidermidis* | 1% | Intracellulaire | 50 |
| *S. epidermidis* | 1% | Extracellulaire | 14 |
| *S. epidermidis* | 5% | Intracellulaire | 50 |
| *S. epidermidis* | 5% | Extracellulaire | 16 |
| CaCl₂ | 0,20% | Intracellulaire | 42 |
| CaCl₂ | 0,20% | Extracellulaire | 57 |
| CaCl₂ | 1% | Intracellulaire | 49 |
| CaCl₂ | 1% | Extracellulaire | 68 |
| CaCl₂ | 5% | Intracellulaire | 69 |
| CaCl₂ | 5% | Extracellulaire | 70 |
| β-D-glucane | 0,2 g/L | Intracellulaire | 83 |
| β-D-glucane | 0,2 g/L | Extracellulaire | 1 |
| β-D-glucane | 0,8 g/L | Intracellulaire | 82 |
| β-D-glucane | 0,8 g/L | Extracellulaire | -1 |
| β-D-glucane | 2,25 g/L | Intracellulaire | 82 |
| β-D-glucane | 2,25 g/L | Extracellulaire | 0 |
| SrCl₂ | 0,69 mg/L | Intracellulaire | 79 |
| SrCl₂ | 0,69 mg/L | Extracellulaire | 0 |
| SrCl₂ | 69,9 mg/L | Intracellulaire | 79 |
| SrCl₂ | 69,9 mg/L | Extracellulaire | 1 |
| SrCl₂ | 699 mg/L | Intracellulaire | 80 |
| SrCl₂ | 699 mg/L | Extracellulaire | 1 |
| SrCl₂ | 6,99 g/L | Intracellulaire | 79 |
| SrCl₂ | 6,99 g/L | Extracellulaire | 7 |
| AlCl₃ | 0,024 g/L | Intracellulaire | 77 |
| AlCl₃ | 0,024 g/L | Extracellulaire | 1 |
| AlCl₃ | 0,121 g/L | Intracellulaire | 80 |
| AlCl₃ | 0,121 g/L | Extracellulaire | 2 |
| AlCl₃ | 0,603 g/L | Intracellulaire | 81 |
| AlCl₃ | 0,603 g/L | Extracellulaire | 26 |
| *L. delbrueckii* | 0,20% | Intracellulaire | 98 |
| *L. delbrueckii* | 0,20% | Extracellulaire | 97 |
| *L. delbrueckii* | 1% | Intracellulaire | 78 |
| *L. delbrueckii* | 1% | Extracellulaire | 86 |
| *L. delbrueckii* | 5% | Intracellulaire | 44 |
| *L. delbrueckii* | 5% | Extracellulaire | 75 |

Seul l'extrait de *Lactobacillus delbrueckii spp lactis* se comporte en éliciteur : près de 100% d'inhibition est observé. C'est un des rares éliciteurs à rendre le milieu de culture très actif (97% d'inhibition en matière sèche équivalente). Enfin, cet éliciteur est très actif à la plus faible concentration soit 0,2%.

Il est à noter que *S. epidermidis* est également une bactérie à Gram positif et qu'elle est sans effet sur la culture d'iris.

## Revendications

1. Utilisation de *Lactobacillus delbrueckii* sous forme d'extrait pour éliciter une cellule végétale d'une Iridacée du genre Romulea, Crocus, Iris, Gladiolus, Sisyrinchium ou Hermodactylus.

2. Utilisation de *Lactobacillus delbrueckii* sous forme d'extrait pour induire la production d'un antagoniste du CGRP par une cellule végétale d'une Iridacée du genre Romulea, Crocus, Iris, Gladiolus, Sisyrinchium ou Hermodactylus.

3. Utilisation selon l'une quelconque des revendications 1 à 2, dans laquelle la cellule végétale est une cellule dédifférenciée d'une Iridacée.

4. Utilisation selon l'une quelconque des revendications 1 à 3, dans laquelle la cellule végétale est une cellule végétale d'Iris.

5. Procédé d'élicitation de cellules végétales d'une Iridacée du genre Romulea, Crocus, Iris, Gladiolus, Sisyrinchium ou Hermodactylus, comprenant la mise en contact de cellules végétales d'une Iridacée du genre Romulea, Crocus, Iris, Gladiolus, Sisyrinchium ou Hermodactylus avec *Lactobacillus delbrueckii* sous forme d'extrait dans des conditions appropriées pour induire l'expression d'une activité antagoniste du CGRP.

6. Procédé de préparation d'une composition ayant une activité antagoniste du CGRP comprenant les étapes consistant à :
a) mettre en contact, dans un milieu de culture, des cellules végétales d'une Iridacée du genre Romulea, Crocus, Iris, Gladiolus, Sisyrinchium ou Hermodactylus avec *Lactobacillus delbrueckii* sous forme d'extrait dans des conditions appropriées pour induire l'expression d'une activité antagoniste du CGRP,
b) éventuellement séparer les cellules végétales cultivées à l'étape a) du milieu de culture, et
c) récupérer une composition ayant une activité antagoniste du CGRP.

7. Composition ayant une activité antagoniste du CGRP susceptible d'être obtenue par le procédé de préparation selon la revendication 6.

8. Composition cosmétique comprenant une composition ayant une activité antagoniste du CGRP selon la revendication 7, et un véhicule cosmétiquement acceptable.

9. Composition cosmétique selon la revendication 8 pour son utilisation dans le traitement des peaux sensibles.

10. Utilisation de la composition cosmétique selon la revendication 8 dans le traitement antipelliculaire.

11. Composition ayant une activité antagoniste du CGRP selon la revendication 7 pour son utilisation dans le traitement des désordres associés à un excès de synthèse et/ou de libération du CGRP.

12. Composition ayant une activité antagoniste du CGRP selon la revendication 7 pour son utilisation selon la revendication 11, ledit désordre associé à un excès de synthèse et/ou de libération du CGRP étant choisi dans le groupe constitué de l'eczéma, le prurigo et la rosacée.

## Patentansprüche

1. Anwendung von *Lactobacillus delbrueckii* in Form eines Extraktes zum Gewinnen einer Pflanzenzelle einer Iridaceae der Gattung Romulea, Crocus, Iris, Gladiolus, Sisyrinchium oder Hermodactylus.

2. Anwendung von *Lactobacillus delbrueckii* in Form eines Extraktes zum Induzieren der Produktion eines Antagonisten zu CGRP mittels einer Pflanzenzelle einer Iridaceae der Gattung Romulea, Crocus, Iris, Gladiolus, Sisyrinchium oder Hermodactylus.

3. Anwendung gemäß einem der Ansprüche 1 bis 2, wobei die Pflanzenzelle eine dedifferenzierte Zelle einer Iridaceae ist.

4. Anwendung gemäß einem der Ansprüche 1 bis 3, wobei die Pflanzenzelle eine Pflanzenzelle einer Iris ist.

5. Verfahren zum Gewinnen von Pflanzenzellen einer Iridaceae der Gattung Romulea, Crocus, Iris, Gladiolus, Sisyrinchium oder Hermodactylus, aufweisend das in Kontakt Bringen von Pflanzenzellen einer Iridaceae der Gattung Romulea, Crocus, Iris, Gladiolus, Sisyrinchium oder Hermodactylus mit einem Extrakt von *Lactobacillus delbrueckii* unter Bedingungen, die geeignet sind, die Expression einer antagonistischen Aktivität zu CGRP zu induzieren.

6. Verfahren zum Herstellen einer Zusammensetzung mit antagonistischer Aktivität zu CGRP, aufweisend die folgenden Schritte:
a) in Kontakt Bringen von Pflanzenzellen einer Iridaceae der Gattung Romulea, Crocus, Iris, Gladiolus, Sisyrinchium oder Hermodactylus mit einem Extrakt von *Lactobacillus delbrueckii* in einem Kulturmedium unter Bedingungen, die geeignet sind, die Expression einer antagonistischen Aktivität zu CGRP zu induzieren,
b) gegebenenfalls Separieren der im Schritt a) kultivierten Pflanzenzellen aus dem Kulturmedium, und
c) Gewinnen einer Zusammensetzung, die eine antagonistische Aktivität zu CGRP aufweist.

7. Zusammensetzung, die eine antagonistische Aktivität zu CGRP aufweist und die mittels des Herstellungsverfahrens gemäß Anspruch 6 gewonnen werden kann.

8. Kosmetische Zusammensetzung, aufweisend eine Zusammensetzung, die eine antagonistische Aktivität zu CGRP aufweist, gemäß Anspruch 7 und einen kosmetisch verträglichen Träger.

9. Kosmetische Zusammensetzung gemäß Anspruch 8 zur Verwendung bei der Behandlung von empfindlicher Haut.

10. Verwendung der kosmetischen Zusammensetzung gemäß Anspruch 8 bei der Antischuppenbehandlung.

11. Zusammensetzung, die eine antagonistische Aktivität zu CGRP aufweist, gemäß Anspruch 7 zur Verwendung bei der Behandlung von Störungen, die mit einem Überschuss an Synthese und/oder Freisetzung von CGRP verbunden sind.

12. Zusammensetzung, die eine antagonistische Aktivität zu CGRP aufweist, gemäß Anspruch 7 zur Verwendung gemäß Anspruch 11, wobei die Störung, die mit einem Überschuss an Synthese und/oder Freisetzung von CGRP verbunden ist, ausgewählt ist aus der Gruppe bestehend aus Ekzem, Prurigo und Rosacea.

## Claims

1. Use of *Lactobacillus delbrueckii* in the form of an extract to elicit a plant cell of an Iridaceae from genera Romulea, Crocus, Iris, Gladiolus, Sisyrinchium or Hermodactylus.

2. Use of *Lactobacillus delbrueckii* in the form of an extract to induce the production of an antagonist of CGRP by a plant cell of an Iridaceae from genera Romulea, Crocus, Iris, Gladiolus, Sisyrinchium or Hermodactylus.

3. Use according to any one of claims 1 to 2, wherein the plant cell is a dedifferentiated cell of an Iridaceae.

4. Use according to any one of claims 1 to 3, wherein the plant cell is an Iris plant cell.

5. Method for elicitation of plant cells of an Iridaceae from genera Romulea, Crocus, Iris, Gladiolus, Sisyrinchium or Hermodactylus comprising placing plant cells of an Iridaceae from genera Romulea, Crocus, Iris, Gladiolus, Sisyrinchium or Hermodactylus in contact with *Lactobacillus delbrueckii* in the form of an extract under conditions suitable for inducing the expression of a CGRP antagonist activity.

6. Method for preparing a composition having a CGRP antagonist activity comprising the steps consisting in:
a) placing in contact, in a culture medium, the plant cells of an Iridaceae from genera Romulea, Crocus, Iris, Gladiolus, Sisyrinchium or Hermodactylus with *Lactobacillus delbrueckii* in the form of an extract under conditions suitable for inducing the expression of a CGRP antagonist activity,
b) optionally separating plant cells cultivated in step a) from the culture medium, and
c) recovering a composition with CGRP antagonist activity.

7. Composition having a CGRP antagonist activity that can be obtained by the method of preparation according to claim 6.

8. Cosmetic composition comprising a composition having a CGRP antagonist activity according to claim 7, and a cosmetically acceptable carrier.

9. Cosmetic composition according to claim 8 for its use in treatment of sensitive skins.

10. Use of the cosmetic composition according to claim 8 in anti-dandruff treatment.

11. Composition having a CGRP antagonist activity according to claim 7 for use in the treatment of disorders associated with an excessive synthesis and/or release of CGRP.

12. Composition having a CGRP antagonist activity according to claim 7 for use according to claim 11, wherein said disorder associated with an excessive synthesis and/or release of CGRP is selected from the group consisting of eczema, prurigo and Rosacea.
